# EUROPÄISCHE PATENTANMELDUNG

(11) **EP 4 049 982 A1**
(43) Veröffentlichungstag der Anmeldung: **31.08.2022**
(21) Anmeldenummer: 21159118.5
(22) Anmeldetag: 24.02.2021
(51) Int. Cl.: C03C 3/097, C03C 4/00, C03C 8/08, C03C 10/00

(54) **GLASKERAMIK MIT QUARZ-MISCHKRISTALLPHASE**

(71) Anmelder: Ivoclar Vivadent AG, 9494 Schaan (LI)
(72) Erfinder: RAMPF, Markus, 7212 Seewis-Dorf (CH); DITTMER, Marc, 6800 Feldkirchen (AT); RITZBERGER, Christian, 9472 Grabs (CH)
(74) Vertreter: Uexküll & Stolberg

(57) **Zusammenfassung**

Es werden Quarz-Mischkristall-Glaskeramiken und Vorstufen davon beschrieben, die sich durch sehr gute mechanische und optische Eigenschaften auszeichnen und insbesondere als Restaurationsmaterial in der Zahnheilkunde eingesetzt werden können.

## Beschreibung

Die Erfindung betrifft Glaskeramik mit Quarz-Mischkristallphase, welche sich insbesondere zum Einsatz in der Zahnheilkunde und bevorzugt zur Herstellung von dentalen Restaurationen eignet, sowie Vorstufen zur Herstellung dieser Glaskeramik.

Glaskeramiken mit Quarz-Mischkristallphase sind aus dem Stand der Technik grundsätzlich bekannt.

Die DE 25 07 131 A1 beschreibt spezielle Magnesium-Aluminosilikat-Glaskeramiken, die 20 bis 35 Gew.-% Al₂O₃ und 9 bis 15 Gew.-% MgO enthalten. Aus den Glaskeramiken hergestellte Körper weisen eine heterogene Struktur auf, indem sich das Kristallgefüge der Oberflächenschicht von dem des Inneren der Körper unterscheidet. Die auf diese Weise erzeugte Oberflächendruckspannung hat einen wesentlichen Einfluss auf die mechanischen Eigenschaften, so dass eine maschinelle Bearbeitung der Oberflächenschicht eine Beeinträchtigung der mechanischen Eigenschaften zur Folge hätte. In der Oberflächenschicht konnten Hochquarz-Mischkristalle und im Inneren der Körper Tiefquarz-Mischkristalle nachgewiesen werden.

Die JP 2000/063144 A offenbart Magnesium-Aluminosilikat-Gläser zur Herstellung von Substraten für Speichermedien, die 30 bis 60 mol-% SiO₂ und große Mengen an B₂O₃ aufweisen.

Die GB 2 172 282 A beschreibt Magnesium-Aluminosilikat-Glaskeramiken, die 10 bis 40 Gew.-% Al₂O₃ enthalten. Die Glaskeramiken sind für mikroelektronische Anwendungen und insbesondere als Beschichtung für Substrate wie z.B. Aluminium vorgesehen und sie haben neben einer hohen Festigkeit eine geeignete Dielektrizitätskonstante im Bereich von 7 bis 10 sowie einen hohen elektrischen Widerstand.

WO 2012/143137 A1 beschreibt glaskeramische Körper, die mindestens 10,1 Gew.-% Al₂O₃ enthalten und in unterschiedlichen Bereichen unterschiedliche Kristallphasen aufweisen.

In dem Artikel von M. Dittmer und C. Rüssel in J. Biomed. Mater. Res. Part B:100B:463-470 (2012) werden Glaskeramiken mit Hochquarz- oder Tiefquarz-Mischkristallphase als Hauptkristallphase beschrieben, die mindestens 25,9 Gew.-% Al₂O₃ enthalten.

Insgesamt sind die mit diesen bekannten Glaskeramiken erzielten Festigkeiten und auch deren optische Eigenschaften nicht völlig zufriedenstellend für eine Anwendung als Dentalmaterial.

Der Erfindung liegt daher die Aufgabe zugrunde, eine Glaskeramik bereitzustellen, die über eine Kombination von hoher Festigkeit und guter Transluzenz verfügt. Die Glaskeramik sollte zudem einen Wärmeausdehnungskoeffizienten aufweisen, der über einen breiten Bereich einstellbar ist. Die Glaskeramik soll weiter in einfacher Weise zu dentalen Restaurationen verarbeitbar sein und sich damit ausgezeichnet als restauratives Dentalmaterial eignen.

Diese Aufgabe wird durch die Glaskeramik mit Quarz-Mischkristallphase nach den Ansprüchen 1 bis 16 und 19 gelöst. Gegenstand der Erfindung sind ebenfalls das Ausgangsglas nach den Ansprüchen 17 bis 19, die Verfahren nach den Ansprüchen 20 und 23 sowie die Verwendung nach den Ansprüchen 21 und 22.

Die erfindungsgemäße Glaskeramik zeichnet sich dadurch aus, dass sie die folgenden Komponenten enthält

| Komponente | Gew. -% |
|---|---|
| SiO₂ | 54,1 bis 67,0 |
| Li₂O | 13,1 bis 18,5 |
| K₂O | 0,1 bis 2,0 |
| Al₂O₃ | 1,6 bis 4, 0 |
| P₂O₅ | 4,1 bis 6,5 |
| ZrO₂ | 7,0 bis 13,5 |

und mindestens eine Quarz-Mischkristallphase enthält.

Diese Glaskeramik, im Folgenden auch als "Glaskeramik mit Quarz-Mischkristallphase" bezeichnet, zeigt überraschenderweise eine vorteilhafte Kombination von für ein restauratives Dentalmaterial wünschenswerten mechanischen und optischen Eigenschaften. Die Glaskeramik hat eine hohe Festigkeit und dennoch kann ihr in einfacher Weise durch Verpressen oder maschinelle Bearbeitung die Form einer Dentalrestauration gegeben werden. Es war weiterhin nicht zu erwarten, dass durch das Vorsehen einer oder mehrerer Quarz-Mischkristallphasen dennoch sehr gute optische Eigenschaften erzielt werden können. Denn viele Nebenkristallphasen haben einen negativen Effekt auf die optischen Eigenschaften von Glaskeramiken. Sie können beispielsweise die Transluzenz vermindern und sie können ebenfalls die Einfärbbarkeit der Glaskeramik beeinträchtigen, was bei der Nachahmung der Farbe des zu ersetzenden natürlichen Zahnmaterials zu erheblichen Schwierigkeiten führen kann.

Weiter hat sich gezeigt, dass sich über Art und Menge der gebildeten Quarz-Mischkristallphase der Wärmeausdehnungskoeffizient der erfindungsgemäßen Glaskeramiken in einem weiten Bereich verändern lässt. Schließlich wurde auch festgestellt, dass die erfindungsgemäßen Glaskeramiken im Vergleich zu Lithiumsilikat-Quarz-Glaskeramiken bei höheren Temperaturen dichtgesintert werden können, ohne dabei ihre Form zu verlieren.

Mit dem Begriff "Quarz-Mischkristallphase" ist eine Kristallphase aus SiO₂ gemeint, bei der in das Gitter des SiO₂ Fremdatome entweder in Zwischengitterplätze oder in Gitterplätze eingebaut sind. Bei diesen Fremdatomen kann es sich insbesondere um Al sowie Li, Mg und/oder Zn handeln. Dabei kann Al vorzugsweise in einer molaren Konzentration vorliegen, die der Summe aus der molaren Konzentration von Li, der Hälfte der molaren Konzentration von Mg und der Hälfte der molaren Konzentration von Zn entspricht.

In einer bevorzugten Ausführungsform enthält die Glaskeramik mindestens zwei unterschiedliche Quarz-Mischkristallphasen.

Bei der mindestens einen Quarz-Mischkristallphase kann es sich sowohl um eine stöchiometrische als auch um eine nicht-stöchiometrische Quarz-Mischkristallphase handeln. Unter stöchiometrischen Quarz-Mischkristallphasen werden solche Kristallphasen verstanden, bei denen die Anzahl der Siliziumatome und die Anzahl eines der Fremdatome im Verhältnis x:y stehen, wobei x und y ganze Zahlen im Bereich von 1 bis 8 und insbesondere im Bereich von 1 bis 5 sind. In einer bevorzugten Ausführungsform enthält die Glaskeramik mindestens eine und vorzugsweise mindestens zwei nicht-stöchiometrische Quarz-Mischkristallphasen.

Bei der mindestens einen Quarz-Mischkristallphase kann es sich um eine stöchiometrische oder nicht-stöchiometrische Aluminosilikat-Kristallphase handeln. In einer bevorzugten Ausführungsform enthält die Glaskeramik mindestens eine und vorzugsweise mindestens zwei stöchiometrische oder nicht-stöchiometrische Aluminosilikat-Kristallphasen. In einer besonders bevorzugten Ausführungsform enthält die Glaskeramik mindestens eine und vorzugsweise mindestens zwei nicht-stöchiometrische Aluminosilikat-Kristallphasen. Dabei werden unter stöchiometrischen Aluminosilikat-Kristallphasen solche Kristallphasen verstanden, bei denen die Anzahl der Siliziumatome und die Anzahl der Aluminiumatome im Verhältnis x:y stehen, wobei x und y ganze Zahlen im Bereich von 1 bis 8 und insbesondere im Bereich von 1 bis 5 sind. Beispiele für stöchiometrische Aluminosilikat-Kristallphasen sind Eucryptit (LiAlSiO₄), Spodumen (LiAlSi₂O₆), Petalit (LiAlSi₄O₁₀) und Cordierit (Mg₂Al₄Si₅O₁₈).

Die Quarz-Mischkristallphasen der erfindungsgemäßen Glaskeramik lassen sich insbesondere durch Röntgenpulverbeugung mittels Cu_{κα}-Strahlung detektieren. Dabei zeigen die Quarz-Mischkristallphasen charakteristische Peakmuster, die sich jeweils vom Peakmuster von Tiefquarz ableiten, aber zu anderen 2Θ-Werten hin verschoben sind.

Die erfindungsgemäße Glaskeramik mit Quarz-Mischkristallphase enthält insbesondere 57,0 bis 66,5, bevorzugt 59,0 bis 66,0 und besonders bevorzugt 62,1 bis 65,5 Gew.-% SiO₂.

Es ist weiter bevorzugt, dass die erfindungsgemäße Glaskeramik 13,3 bis 18,0, bevorzugt 16,1 bis 17,5 und besonders bevorzugt 16,5 bis 17,0 Gew.-% Li₂O enthält. Es wird angenommen, dass Li₂O die Viskosität der Glasmatrix erniedrigt und damit die Kristallisation der gewünschten Phasen fördert.

Auch ist es bevorzugt, dass die Glaskeramik 0,5 bis 1,7, vorzugsweise 1,1 bis 1,5 und besonders bevorzugt 1,2 bis 1,4 Gew.-% K₂O enthält.

In einer bevorzugten Ausführungsform enthält die erfindungsgemäße Glaskeramik 2,0 bis 3,8 und bevorzugt 1,6 bis 3,6 Gew.-% Al₂O₃. In einer weiteren bevorzugten Ausführungsform enthält die Glaskeramik 4,3 bis 6,0, bevorzugt 4,5 bis 5,9 und besonders bevorzugt 5,1 bis 5,8 Gew.-% P₂O₅. Es wird angenommen, dass das P₂O₅ als Keimbildner wirkt.

Es ist weiter bevorzugt, dass die Glaskeramik 7,2 bis 13,0 und bevorzugt 9,0 bis 12,0 Gew.-% ZrO₂ enthält.

Auch ist es bevorzugt, dass die Glaskeramik 1,0 bis 8,0, bevorzugt 1,0 bis 5,5 und besonders bevorzugt 1,5 bis 2,5 Gew.-% Oxid einwertiger Elemente Me^{I}₂O ausgewählt aus der Gruppe von Na₂O, Rb₂O, Cs₂O und Mischungen davon enthält.

Besonders bevorzugt enthält die Glaskeramik mindestens eines und insbesondere alle der folgenden Oxide einwertiger Elemente Me^{I}₂O in den angegebenen Mengen:

| Komponente | Gew.-% |
|---|---|
| Na₂O | 0 bis 2,0 |
| Rb₂O | 0 bis 8,0 |
| Cs₂O | 0 bis 7,0. |

Die erfindungsgemäße Glaskeramik enthält vorzugsweise 0,05 bis 5,0, insbesondere 0,07 bis 1,5, bevorzugt 0,08 bis 1,0, besonders bevorzugt 0,09 bis 0,4 und ganz besonders bevorzugt 0,1 bis 0,2 Gew.-% Oxid zweiwertiger Elemente Me^{II}O ausgewählt aus der Gruppe von CaO, MgO, SrO, ZnO und Mischungen davon, wobei das Me^{II}O vorzugsweise MgO ist. Es wird angenommen, dass Oxide zweiwertiger Elemente Me^{II}O und insbesondere MgO die Bildung von einer oder mehreren Quarz-Mischkristallphasen fördern und die Bildung von unerwünschten Kristallphasen wie insbesondere Cristobalit vermeiden, die nachteiligen Einfluss auf den Wärmeausdehnungskoeffizienten und die optischen Eigenschaften haben können.

In einer weiteren bevorzugten Ausführungsform enthält die Glaskeramik weniger als 2,0 Gew.-% an BaO. Die Glaskeramik ist insbesondere im Wesentlichen frei von BaO.

Bevorzugt enthält die Glaskeramik mindestens eines und insbesondere alle der folgenden Oxide zweiwertiger Elemente Me^{II}O in den angegebenen Mengen:

| Komponente | Gew.-% |
|---|---|
| CaO | 0 bis 3,0 |
| MgO | 0 bis 5,0 |
| SrO | 0 bis 5,0 |
| ZnO | 0 bis 3,0 |

Es ist weiter eine Glaskeramik bevorzugt, die 0 bis 5,0, bevorzugt 1,0 bis 4,0 und besonders bevorzugt 2,0 bis 3,0 Gew.-% Oxid dreiwertiger Elemente Me^{III}₂O₃ ausgewählt aus der Gruppe von B₂O₃, Y₂O₃, La₂O₃, Ga₂O₃, In₂O₃ und Mischungen davon enthält.

Besonders bevorzugt enthält die Glaskeramik mindestens eines und insbesondere alle der folgenden Oxide dreiwertiger Elemente M2^{III}₂O₃ in den angegebenen Mengen:

| Komponente | Gew.-% |
|---|---|
| B₂O₃ | 0 bis 4, 0 |
| Y₂O₃ | 0 bis 5, 0 |
| La₂O₃ | 0 bis 5, 0 |
| Ga₂O₃ | 0 bis 3, 0 |
| In₂O₃ | 0 bis 5, 0 |

Ferner ist eine Glaskeramik bevorzugt, die 0 bis 10, 0 und besonders bevorzugt 0 bis 8,0 Gew.-% Oxid vierwertiger Elemente Me^{IV}O₂ ausgewählt aus der Gruppe von TiO₂, SnO₂, CeO₂, GeO₂ und Mischungen davon enthält.

Besonders bevorzugt enthält die Glaskeramik mindestens eines und insbesondere alle der folgenden Oxide vierwertiger Elemente Me^{IV}O₂ in den angegebenen Mengen:

| Komponente | Gew.-% |
|---|---|
| TiO₂ | 0 bis 4,0 |
| SnO₂ | 0 bis 3,0 |
| GeO₂ | 0 bis 9,0, insbesondere 0 bis 8,0 |
| CeO₂ | 0 bis 4,0. |

In einer weiteren Ausführungsform enthält die Glaskeramik 0 bis 8,0, bevorzugt 0 bis 6,0 Gew.-% Oxid fünfwertiger Elemente Me^{V}₂O₅ ausgewählt aus der Gruppe von aus V₂O₅, Ta₂O₅, Nb₂O₅ und Mischungen davon.

Besonders bevorzugt enthält die Glaskeramik mindestens eines und insbesondere alle der folgenden Oxide fünfwertiger Elemente Me^{V}₂O₅ in den angegebenen Mengen:

| Komponente | Gew.-% |
|---|---|
| V₂O₅ | 0 bis 2, 0 |
| Ta₂O₅ | 0 bis 5, 0 |
| Nb₂O₅ | 0 bis 5, 0 |

In einer weiteren Ausführungsform enthält die Glaskeramik 0 bis 5,0, bevorzugt 0 bis 4,0 Gew.-% Oxid sechswertiger Element Me^{VI}O₃ ausgewählt aus der Gruppe von WO₃, MoO₃ und Mischungen davon.

Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 3,0 |
| MoO₃ | 0 bis 3,0 |

In einer weiteren Ausführungsform enthält die erfindungsgemäße Glaskeramik 0 bis 1,0 und insbesondere 0 bis 0,5 Gew.-% Fluor.

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 54,1 bis 67,0 |
| Li₂O | 13, 1 bis 18,5 |
| K₂O | 0, 1 bis 2, 0 |
| Al₂O₃ | 1,6 bis 4,0 |
| P₂O₅ | 4,1 bis 6,5 |
| ZrO₂ | 7,0 bis 13,5 |
| Me^{I}₂O | 1,0 bis 8,0 |
| Me^{II}O | 0 bis 5, 0 |
| Me^{III}₂O₃ | 1,0 bis 8,0 |
| Me^{IV}O₂ | 0 bis 10,0 |
| Me^{V}₂O₅ | 0 bis 8,0 |
| Me^{VI}O₃ | 0 bis 5, 0 |
| Fluor | 0 bis 1,0, |

wobei Me^{I}₂O, Me^{II}O, Me^{III}₂O₃, Me^{IV}O₂, Me^{V}₂O₅ und Me^{VI}O₃ die oben angegebene Bedeutung haben.

In einer weiteren besonders bevorzugten Ausführungsform enthält die Glaskeramik mindestens eine und bevorzugt alle der folgenden Komponenten in den angegebenen Mengen:

| Komponente | Gew. - % |
|---|---|
| SiO₂ | 54,1 bis 67,0 |
| Li₂O | 13,1 bis 18,5 |
| K₂O | 0,1 bis 2,0 |
| Al₂O₃ | 1,6 bis 4, 0 |
| P₂O₅ | 4,1 bis 6,5 |
| ZrO₂ | 7,0 bis 13,5 |
| Na₂O | 0 bis 2,0 |
| Rb₂O | 0 bis 8,0 |
| Cs₂O | 0 bis 7,0 |
| CaO | 0 bis 3,0 |
| MgO | 0 bis 5,0 |
| SrO | 0 bis 5,0 |
| ZnO | 0 bis 3,0 |
| B₂O₃ | 0 bis 4,0 |
| Y₂O₃ | 0 bis 5,0 |
| La₂O₃ | 0 bis 5,0 |
| Ga₂O₃ | 0 bis 3,0 |
| In₂O₃ | 0 bis 5,0 |
| TiO₂ | 0 bis 4,0 |
| SnO₂ | 0 bis 3,0 |
| GeO₂ | 0 bis 9,0, insbesondere 0 bis 8,0 |
| CeO₂ | 0 bis 4,0 |
| V₂O₅ | 0 bis 2,0 |
| Ta₂O₅ | 0 bis 5,0 |
| Nb₂O₅ | 0 bis 5,0 |
| WO₃ | 0 bis 3,0 |
| MoO₃ | 0 bis 3,0 |
| Fluor | 0 bis 1,0. |

Manche der vorstehend genannten Komponenten können als Färbemittel und/oder Fluoreszenzmittel dienen. Die erfindungsgemäße Glaskeramik kann darüber hinaus noch weitere Färbemittel und/oder Fluoreszenzmittel enthalten. Diese können z.B. aus Bi₂O₃ oder Bi₂O₅ und insbesondere aus weiteren anorganischen Pigmenten und/oder Oxiden von d- und f-Elementen, wie z.B. den Oxiden von Mn, Fe, Co, Pr, Nd, Tb, Er, Dy, Eu und Yb, ausgewählt sein. Mithilfe dieser Färbemittel und Fluoreszenzmittel ist eine einfache Einfärbung der Glaskeramik möglich, um die gewünschten optischen Eigenschaften insbesondere von natürlichem Zahnmaterial zu imitieren. Es ist überraschend, dass dies trotz des Vorhandenseins einer oder mehrerer Quarz-Mischkristallphasen problemlos möglich ist.

In einer bevorzugten Ausführungsform der Glaskeramik liegt das Molverhältnis von SiO₂ zu Li₂O im Bereich von im Bereich von 1,5 bis 6, 0, insbesondere 1,55 bis 3, 0, bevorzugt 1,6 bis 1,8 und besonders bevorzugt 1,65 bis 1,75. Es ist überraschend, dass innerhalb dieser breiten Bereiche die Herstellung der erfindungsgemäßen Glaskeramik mit Quarz-Mischkristallphase gelingt.

Es ist weiter bevorzugt, dass die erfindungsgemäße Glaskeramik Lithiumdisilikat oder Lithiummetasilikat als weitere Kristallphasen und insbesondere als Hauptkristallphase enthält. Besonders bevorzugt enthält die erfindungsgemäße Glaskeramik Lithiumdisilikat als weitere Kristallphasen und insbesondere als Hauptkristallphase.

Mit dem Begriff "Hauptkristallphase" wird die Kristallphase bezeichnet, die von allen in der Glaskeramik vorhandenen Kristallphasen den höchsten Gewichtsanteil hat. Die Bestimmung der Mengen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Es ist bevorzugt, dass die erfindungsgemäße Glaskeramik mindestens 20 Gew.-%, bevorzugt 25 bis 55 Gew.-% und besonders bevorzugt 30 bis 55 Gew.-% Lithiumdisilikat-Kristalle aufweist.

Es ist weiter bevorzugt, dass die erfindungsgemäße Glaskeramik 0,2 bis 28 Gew.-% und bevorzugt 0,2 bis 25 Gew.-% Quarz-Mischkristalle aufweist.

Die erfindungsgemäße Glaskeramik mit Quarz-Mischkristallphase zeichnet sich durch besonders gute mechanische Eigenschaften und optische Eigenschaften aus und sie kann durch Wärmebehandlung eines entsprechenden Ausgangsglases oder eines entsprechenden Ausgangsglases mit Keimen gebildet werden. Diese Materialien können daher als Vorstufen für die erfindungsgemäße Glaskeramik mit Quarz-Mischkristallphase dienen.

Die Art und insbesondere die Menge der gebildeten Kristallphasen können durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung des Ausgangsglases und der angewendeten Wärmebehandlung.

Die Glaskeramik weist eine hohe biaxiale Bruchfestigkeit von vorzugsweise mindestens 200 MPa und besonders bevorzugt 250 bis 460 MPa auf. Die biaxiale Bruchfestigkeit wurde gemäß ISO 6872 (2008) (Kolben-auf-drei-Kugeln-Prüfung) bestimmt.

Die erfindungsgemäße Glaskeramik weist insbesondere einen thermischen Ausdehnungskoeffizienten WAK (gemessen im Bereich von 100 bis 500°C) von 3,0 bis 14,0.10⁻⁶ K⁻¹, bevorzugt 5,0 bis 14,0.10⁻⁶ K⁻¹ und besonders bevorzugt 7,0 bis 14,0.10⁻⁶ K⁻¹ auf. Der WAK wird gemäß ISO 6872 (2008) bestimmt. Eine Einstellung des Wärmeausdehnungskoeffizienten auf einen gewünschten Wert erfolgt insbesondere durch die Art und Menge der in der Glaskeramik vorhandenen Kristallphasen sowie die chemische Zusammensetzung der Glaskeramik.

Die Transluzenz der Glaskeramik wurde in Form des Kontrastwerts (CR-Wert) gemäß British Standard BS 5612 bestimmt, und dieser Kontrastwert betrug vorzugsweise 40 bis 92.

Die bei der erfindungsgemäßen Glaskeramik vorliegende besondere Kombination an Eigenschaften erlaubt es sogar, sie als Dentalmaterial und insbesondere als Material zur Herstellung von Dentalrestaurationen einzusetzen.

Die Erfindung betrifft ebenfalls Vorstufen mit entsprechender Zusammensetzung, aus denen die erfindungsgemäße Glaskeramik mit Quarz-Mischkristallphase durch Wärmebehandlung hergestellt werden können. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten wie bei Gläsern und Glaskeramiken üblich mit Ausnahme von Fluor als Oxide berechnet werden.

Die Erfindung betrifft daher ebenfalls ein Ausgangsglas, das die Komponenten der erfindungsgemäßen Glaskeramik mit Quarz-Mischkristallphase enthält.

Das erfindungsgemäße Ausgangsglas enthält daher insbesondere geeignete Mengen an SiO₂, Li₂O, K₂O, Al₂O₃, P₂O₅ und ZrO₂, die zur Ausbildung der erfindungsgemäßen Glaskeramik mit Quarz-Mischkristallphase erforderlich sind. Weiter kann das Ausgangsglas auch noch andere Komponenten enthalten, wie sie oben für die erfindungsgemäße Glaskeramik mit Quarz-Mischkristallphase angegeben sind. Es sind alle solche Ausführungsformen für die Komponenten des Ausgangsglases bevorzugt, die auch für die Komponenten der erfindungsgemäßen Glaskeramik mit Quarz-Mischkristallphase als bevorzugt angegeben sind.

Besonders bevorzugt liegt das Ausgangsglas in Form eines Pulvers, eines Granulats oder eines aus einem Pulver oder Granulat gepressten Pulverpresslings vor. Im Gegensatz zu einem Glasmonolithen, wie er etwa durch Gießen einer Glasschmelze in eine Form erhalten wird, hat das Ausgangsglas in den genannten Formen eine große innere Oberfläche, an denen die spätere Kristallisation von einer und bevorzugt mehreren Quarz-Mischkristallphasen erfolgen kann.

Die Erfindung betrifft ebenfalls ein solches Ausgangsglas, das Keime für die Kristallisation von Quarz-Mischkristallphase enthält. Vorzugsweise enthält das Ausgangsglas ferner Keime für die Kristallisation von Lithiumdisilikat oder Lithiummetasilikat.

Die Herstellung des Ausgangsglases erfolgt insbesondere in der Weise, dass eine Mischung von geeigneten Ausgangsmaterialien, wie z.B. Carbonaten und Oxiden, bei Temperaturen von insbesondere etwa 1500 bis 1700°C für 0,5 bis 4 h geschmolzen wird. Zur Erzielung einer besonders hohen Homogenität kann die erhaltene Glasschmelze in Wasser gegossen werden, um eine Glasfritte zu bilden, und die erhaltene Fritte wird dann erneut aufgeschmolzen.

Die Schmelze kann dann in Formen, z.B. Stahl- oder Graphitformen, gegossen werden, um Rohlinge des Ausgangsglases, sogenannte Massivglasrohlinge oder monolithische Rohlinge, zu erzeugen. Üblicherweise werden diese monolithischen Rohlinge zunächst entspannt, z.B. indem sie 5 bis 60 min bei 800 bis 1200°C gehalten werden, und dann langsam auf Raumtemperatur abgekühlt.

In einer bevorzugten Ausführungsform wird die Schmelze in Wasser gegossen, um eine Fritte herzustellen. Diese Glasfritte kann durch Mahlen zu einem Pulver oder Granulat verarbeitet werden. Vorzugsweise kann das so erhaltene Pulver oder Granulat, gegebenenfalls nach Zugabe weiterer Komponenten, wie Färbe- und Fluoreszenzmitteln, zu einem Rohling, einem sogenannten Pulverpressling, gepresst werden. Dabei können durch Verwendung von mehreren unterschiedlich eingefärbten Pulvern in einfacher Weise mehrfarbige Rohlinge erhalten werden, die mehrere Bereiche mit unterschiedlichen Farbeigenschaften aufweisen. Dadurch ermöglicht die Erfindung die Herstellung von hochästhetischen mehrfarbigen dentalen Restaurationen, welche die optischen Eigenschaften des natürlichen Zahnmaterials besonders gut imitieren können.

Durch Wärmebehandlung des Ausgangsglases kann zunächst die weitere Vorstufe Ausgangsglas mit Keimen erzeugt werden. Durch Wärmebehandlung dieser weiteren Vorstufe kann dann die erfindungsgemäße Glaskeramik mit Quarz-Mischkristallphase erzeugt werden. Alternativ kann die erfindungsgemäße Glaskeramik mit Quarz-Mischkristallphase durch Wärmebehandlung des Ausgangsglases gebildet werden.

Es ist bevorzugt, das Ausgangsglas einer Wärmebehandlung bei einer Temperatur von 400 bis 600°C, insbesondere 450 bis 550°C, für eine Dauer von insbesondere 5 bis 120 min, bevorzugt 10 bis 60 min, zu unterwerfen, um das Ausgangsglas mit Keimen für die Kristallisation von Quarz-Mischkristallphase zu erzeugen.

Es ist weiter bevorzugt, das Ausgangsglas oder das Ausgangsglas mit Keimen mindestens einer Wärmebehandlung bei einer Temperatur von 600 bis 1000°C, vorzugsweise 650 bis 900°C und besonders bevorzugt 750 bis 900°C, für eine Dauer von insbesondere 1 bis 240 min, bevorzugt 5 bis 120 min und besonders bevorzugt 10 bis 60 min, zu unterwerfen, um die Glaskeramik mit Quarz-Mischkristallphase herzustellen. In einer besonders bevorzugten Ausführungsform wird das Ausgangsglas oder das Ausgangsglas mit Keimen einer ersten Wärmebehandlung bei einer Temperatur von 600 bis 800°C, vorzugsweise 650 bis 750°C und besonders bevorzugt 650 bis 700°C, für eine Dauer von insbesondere 1 bis 120 min, bevorzugt 5 bis 120 min und besonders bevorzugt 10 bis 60 min, und danach einer zweiten Wärmebehandlung bei einer Temperatur von 750 bis 950°C, vorzugsweise 800 bis 900°C und besonders bevorzugt 800 bis 850°C, für eine Dauer von insbesondere 1 bis 120 min, bevorzugt 5 bis 120 min und besonders bevorzugt 10 bis 60 min, unterworfen.

Die Erfindung betrifft daher ebenfalls ein Verfahren zur Herstellung der erfindungsgemäßen Glaskeramik mit Quarz-Mischkristallphase, bei dem das Ausgangsglas oder das Ausgangsglas mit Keimen, insbesondere in partikulärer Form, bevorzugt in Form eines Pulvers und besonders bevorzugt in Form eines Pulverpresslings, mindestens einer Wärmebehandlung im Bereich von 600 bis 1000°C, vorzugsweise 650 bis 900°C, für eine Dauer von insbesondere 1 bis 240 min, bevorzugt 5 bis 120 min und besonders bevorzugt 10 bis 60 min, unterzogen und insbesondere gesintert wird.

Die im erfindungsgemäßen Verfahren durchgeführte mindestens eine Wärmebehandlung kann auch im Rahmen eines Heißpressens oder Aufsinterns des erfindungsgemäßen Ausgangsglases oder des erfindungsgemäßen Ausgangsglases mit Keimen erfolgen.

Die erfindungsgemäßen Glaskeramiken und die erfindungsgemäßen Gläser liegen insbesondere als Pulver, Granulate oder Rohlinge in beliebiger Form und Größe, z.B. monolithische Rohlingen, wie Plättchen, Quader oder Zylinder, oder Pulverpresslinge, vor. In diesen Formen können sie einfach weiterverarbeitet werden, z.B. zu dentalen Restaurationen. Sie können aber auch in Form von dentalen Restaurationen, wie Inlays, Onlays, Kronen, Veneers, Schalen oder Abutments, vorliegen.

Besonders bevorzugt liegen die erfindungsgemäßen Glaskeramiken in Form von mehrfarbigen Rohlingen, insbesondere mehrfarbigen vorgesinterten oder gesinterten Pulverpresslingen, vor.

Aus den erfindungsgemäßen Glaskeramiken und den erfindungsgemäßen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen, insbesondere mehrfarbiger dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas, das erfindungsgemäße Ausgangsglas mit Keimen und die erfindungsgemäße Glaskeramik mit Quarz-Mischkristallphase verwendet werden. Dabei können die erfindungsgemäßen Gläser und Glaskeramiken insbesondere in Form von Rohlingen in beliebiger Form und Größe, z.B. Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Ausgangsglas mit Keimen und die erfindungsgemäße Glaskeramik mit Quarz-Mischkristallphase verwendet werden. Dabei können die erfindungsgemäßen Gläser und Glaskeramiken insbesondere in Form von Rohlingen, z.B. Pulverpresslingen, z.B. in ungesinterter, teilgesinterter oder dichtgesinterter Form, eingesetzt werden.

Nach der Herstellung der gewünscht geformten dentalen Restauration, z.B. durch Verpressen oder maschinelle Bearbeitung, kann diese noch wärmebehandelt werden, um die Porosität, z.B. eines eingesetzten porösen Pulverpresslings, zu vermindern.

Die erfindungsgemäßen Glaskeramiken und die erfindungsgemäßen Gläser eignen sich allerdings auch als Beschichtungsmaterial von z.B. Keramiken und Glaskeramiken. Die Erfindung ist daher ebenfalls auf die Verwendung der erfindungsgemäßen Gläser oder der erfindungsgemäßen Glaskeramiken zur Beschichtung von insbesondere Keramiken und Glaskeramiken gerichtet.

Die Erfindung betrifft auch ein Verfahren zur Beschichtung von Keramiken, Metallen, Metalllegierungen und Glaskeramiken, bei dem erfindungsgemäße Glaskeramik oder erfindungsgemäßes Glas auf das entsprechende Substrat aufgebracht und erhöhter Temperatur ausgesetzt wird.

Dies kann insbesondere durch Aufsintern oder durch Fügen eines mittels CAD-CAM hergestellten Überwurfs mit einem geeigneten Glaslot oder Kleber und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder erfindungsgemäßes Glas, z.B. in Form von Pulverpresslingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial, bevorzugt zur Beschichtung dentaler Restaurationen und besonders bevorzugt zur Herstellung dentaler Restaurationen, wie Brücken, Inlays, Onlays, Veneers, Abutments, Teilkronen, Kronen oder Schalen.

Die Erfindung betrifft mithin auch ein Verfahren zur Herstellung einer dentalen Restauration, insbesondere Brücke, Inlay, Onlay, Veneer, Abutment, Teilkrone, Krone oder Schale, bei dem der erfindungsgemäßen Glaskeramik oder dem erfindungsgemäßen Glas durch Verpressen oder durch maschinelle Bearbeitung, insbesondere im Rahmen eines CAD/CAM-Verfahrens, die Form der gewünschten dentalen Restauration gegeben wird. Bevorzugt handelt es sich dabei um eine mehrfarbige dentale Restauration. Mit einer solchen Restauration können die optischen Eigenschaften des natürlichen Zahnmaterials besonders gut nachgeahmt werden.

Die Erfindung wird im Folgenden anhand von sie nicht-beschränkenden Beispielen näher erläutert.

### Beispiele

### Beispiele 1 bis 7 - Zusammensetzung und Kristallphasen

Es wurden insgesamt 7 erfindungsgemäße Gläser und Glaskeramiken mit der aus Tabelle I angegebenen Zusammensetzung über Erschmelzung entsprechender Ausgangsgläser und anschließende Wärmebehandlung zur gesteuerten Kristallisation hergestellt.

Die angewendeten Wärmebehandlungen sind ebenfalls in Tabelle I angegeben. Dabei bedeuten
- T_{g}: Glasübergangstemperatur, bestimmt mittels DSC
- T_{Kb} und t_{Kb}: Angewendete Temperatur und Zeit für Keimbildung des Ausgangsglases
- T_{C} und t_{C}: Angewendete Temperatur und Zeit für die Kristallisation

Dazu wurden zunächst die Ausgangsgläser aus üblichen Rohstoffen in einem Platin-Rhodium-Tiegel bei 1500 bis 1700°C erschmolzen.

In den Beispielen 1 bis 3 wurden die Schmelzen der Ausgangsgläser in Graphit- oder Stahlformen eingegossen, um Glasmonolithe zu erzeugen. Diese Glasmonolithe wurden entspannt und langsam auf Raumtemperatur abgekühlt. Anschließend wurden sie einer ersten Wärmebehandlung bei der Temperatur T_{Kb} für eine Dauer t_{Kb} zur Keimbildung und danach einer weiteren Wärmebehandlung bei der Temperatur T_{C} für eine Dauer t_{C} zur Kristallisation unterworfen.

In den Beispielen 4 bis 7 wurden durch Eingießen der erschmolzenen Ausgangsgläser in Wasser Glasfritten, d.h. Glasgranulate, hergestellt. Die Glasfritten wurden unter Verwendung von Kugel- oder Mörsermühlen auf eine Partikelgröße von < 45 µm aufgemahlen und mittels Pulverpressen zu Pulverpresslingen verpresst. Die Pulverpresslinge wurden zur Keimbildung und Kristallisation einer ersten Wärmebehandlung bei der Temperatur T_{C1} für eine Dauer t_{C1} und einer zweiten Wärmebehandlung bei der Temperatur T_{C2} für eine Dauer t_{C2} unterworfen.

In der folgenden Tabelle I bedeutet:
- QMK:: Quarz-Mischkristallphase
- SP: Spodumen (LiAlSi₂O₆)

**Tabelle I**

| Beispiel | 1 | 2 | 3 |
|---|---|---|---|
| Zusammensetzung | Gew. - % | Gew. - % | Gew. - % |
| SiO₂ | 62,5 | 65,0 | 66,1 |
| Li₂O | 14,2 | 13,3 | 14,5 |
| K₂O | 1,4 | 0,5 | 1,4 |
| Al₂O₃ | 2,4 | 3,6 | 2, 0 |
| P₂O₅ | 5,4 | 4,5 | 5, 8 |
| ZrO₂ | 10,1 | 13,0 | 7,2 |
| MgO | 3,0 | 0, 1 | 0,5 |
| B₂O₃ | 1,0 | - | 2,5 |
| T_{g} [°C] | 490 | 531 | 488 |
| T_{Kb} [°C] | 510 | 550 | 510 |
| T_{Kb} [min] | 10 | 10 | 10 |
| T_{C} [°C] | 800 | 800 | 810 |
| T_{C} [min] | 30 | 30 | 30 |
| Kristallphasen | Li₂Si₂O₅ QMK | Li₂Si₂O₅ QMK | Li₂Si₂O₅ QMK:SP |

**Tabelle I (Fortsetzung)**

| **Beispiel** | **4** | **5** | **6** | **7** |
|---|---|---|---|---|
| Zusammensetzung | Gew.-% | Gew.-% | Gew.-% | Gew.-% |
| SiO₂ | 59,0 | 60,0 | 62,0 | 63,0 |
| Li₂O | 16,0 | 17,5 | 16,0 | 14,2 |
| K₂O | 1,3 | 1,0 | 1,5 | 1,2 |
| Al₂O₃ | 2,8 | 2,8 | 3,4 | 3,8 |
| P₂O₅ | 5,6 | 6,0 | 5,9 | 5,8 |
| ZrO₂ | 10,0 | 9,5 | 10,5 | 11,5 |
| MgO | 0,1 | 1,5 | 0,2 | 0,4 |
| B₂O₃ | 2,5 | 1,7 | 0,5 | 0,1 |
| Y₂O₃ | 0,1 | - | - | - |
| Ce₂O₃ | 1,0 | - | - | - |
| V₂O₅ | 0,4 | - | - | - |
| Er₂O₃ | 0,2 | - | - | - |
| Tb₄O₇ | 1,0 | - | - | - |
| T_{g} [°C] | 488 | 475 | 499 | 524 |
| T_{C1} [°C] | 750 | 750 | 750 | 750 |
| T_{C1} [min] | 5 | 5 | 5 | 5 |
| T_{C2} [°C] | 880 | 880 | 880 | 880 |
| T_{C2} [min] | 10 | 10 | 10 | 10 |

## Patentansprüche

1. Glaskeramik, die die folgenden Komponenten
| Komponente | Gew.-% |
|---|---|
| SiO₂ | 54,1 bis 67,0 |
| Li₂O | 13,1 bis 18,5 |
| K₂O | 0, 1 bis 2, 0 |
| Al₂O₃ | 1,6 bis 4,0 |
| P₂O₅ | 4,1 bis 6,5 |
| ZrO₂ | 7,0 bis 13,5 |
und mindestens eine Quarz-Mischkristallphase enthält.

2. Glaskeramik nach Anspruch 1, die mindestens zwei unterschiedliche Quarz-Mischkristallphasen enthält.

3. Glaskeramik nach Anspruch 1 oder 2, die mindestens eine und vorzugsweise mindestens zwei stöchiometrische oder nicht-stöchiometrische Aluminosilikat-Kristallphasen und besonders bevorzugt mindestens eine und vorzugsweise mindestens zwei nicht-stöchiometrische Aluminosilikat-Kristallphasen enthält.

4. Glaskeramik nach einem der Ansprüche 1 bis 3, die 57,0 bis 66,5, bevorzugt 59,0 bis 66,0 und besonders bevorzugt 62,1 bis 65,5 Gew.-% SiO₂ enthält.

5. Glaskeramik nach einem der Ansprüche 1 bis 4, die 13,3 bis 18,0, bevorzugt 16,1 bis 17,5 und besonders bevorzugt 16,5 bis 17,0 Gew.-% Li₂O enthält.

6. Glaskeramik nach einem der Ansprüche 1 bis 5, die 0,5 bis 1,7, bevorzugt 1,1 bis 1,5 und besonders bevorzugt 1,2 bis 1,4 Gew.-% K₂O enthält.

7. Glaskeramik nach einem der Ansprüche 1 bis 6, die 2,0 bis 3,8 und bevorzugt 1,6 bis 3,6 Gew.-% Al₂O₃ enthält.

8. Glaskeramik nach einem der Ansprüche 1 bis 7, die 4,3 bis 6,0, bevorzugt 4,5 bis 5,9 und besonders bevorzugt 5,1 bis 5,8 Gew.-% P₂O₅ enthält.

9. Glaskeramik nach einem der Ansprüche 1 bis 8, die 7,2 bis 13,0, und bevorzugt 9,0 bis 12,0 Gew.-% ZrO₂ enthält.

10. Glaskeramik nach einem der Ansprüche 1 bis 9, die 1,0 bis 8,0, bevorzugt 1,0 bis 5,5 und besonders bevorzugt 1,5 bis 2,5 Gew.-% Oxid einwertiger Elemente Me^{I}₂O ausgewählt aus der Gruppe von Na₂O, Rb₂O, Cs₂O und Mischungen davon enthält.

11. Glaskeramik nach einem der Ansprüche 1 bis 10, die 0,05 bis 5,0, insbesondere 0,07 bis 1,5, bevorzugt 0,08 bis 1,0, besonders bevorzugt 0,09 bis 0,4 und ganz besonders bevorzugt 0,1 bis 0,2 Gew.-% Oxid zweiwertiger Elemente Me^{II}O ausgewählt aus der Gruppe von CaO, MgO, SrO, ZnO und Mischungen davon enthält, wobei das Me^{II}O vorzugsweise MgO ist.

12. Glaskeramik nach einem der Ansprüche 1 bis 11, die 0 bis 5,0, bevorzugt 1,0 bis 4,0 und besonders bevorzugt 2,0 bis 3,0 Gew.-% Oxid dreiwertiger Elemente Me^{III}₂O₃ ausgewählt aus der Gruppe von B₂O₃, Y₂O₃, La₂O₃, Ga₂O₃, In₂O₃ und Mischungen davon enthält.

13. Glaskeramik nach einem der Ansprüche 1 bis 12, die SiO₂ und Li₂O in einem Molverhältnis im Bereich von 1,5 bis 6,0, insbesondere 1,55 bis 3,0, bevorzugt 1,6 bis 1,8 und besonders bevorzugt 1,65 bis 1,75 enthält.

14. Glaskeramik nach einem der Ansprüche 1 bis 13, die Lithiumdisilikat oder Lithiummetasilikat als Hauptkristallphase und bevorzugt Lithiumdisilikat als Hauptkristallphase enthält.

15. Glaskeramik nach einem der Ansprüche 1 bis 14, die mindestens 20 Gew.-%, bevorzugt 25 bis 55 Gew.-% und besonders bevorzugt 30 bis 55 Gew.-% Lithiumdisilikat-Kristalle aufweist.

16. Glaskeramik nach einem der Ansprüche 1 bis 15, die 0,2 bis 28 Gew.-% und bevorzugt 0,2 bis 25 Gew.-% Quarz-Mischkristalle aufweist.

17. Ausgangsglas, das die Komponenten der Glaskeramik nach einem der Ansprüche 1 bis 13 enthält.

18. Ausgangsglas nach Anspruch 17, das Keime für die Kristallisation von Quarz-Mischkristallphase und vorzugsweise ferner Keime für die Kristallisation von Lithiumdisilikat oder Lithiummetasilikat enthält.

19. Glaskeramik nach einem der Ansprüche 1 bis 16 oder Ausgangsglas nach Anspruch 17 oder 18, wobei die Glaskeramik und das Ausgangsglas in Form von einem Pulver, einem Granulat, einem Rohling oder einer dentalen Restauration vorliegen.

20. Verfahren zur Herstellung der Glaskeramik gemäß einem der Ansprüche 1 bis 16, bei dem das Ausgangsglas gemäß Anspruch 17 oder 18, insbesondere in partikulärer Form, bevorzugt in Form eines Pulvers und besonders bevorzugt in Form eines Pulverpresslings, mindestens einer Wärmebehandlung im Bereich von 600 bis 1000°C, vorzugsweise 650 bis 900°C, unterzogen und insbesondere gesintert wird.

21. Verwendung der Glaskeramik gemäß einem der Ansprüche 1 bis 16 oder 19 oder des Ausgangsglases gemäß einem der Ansprüche 17 bis 19 als Dentalmaterial, bevorzugt zur Beschichtung dentaler Restaurationen und besonders bevorzugt zur Herstellung dentaler Restaurationen.

22. Verwendung zur Herstellung dentaler Restaurationen nach Anspruch 21, wobei der Glaskeramik oder dem Ausgangsglas durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

23. Verfahren zur Herstellung einer dentalen Restauration, insbesondere Brücke, Inlay, Onlay, Veneer, Abutment, Teilkrone, Krone oder Schale, bei dem der Glaskeramik gemäß einem der Ansprüche 1 bis 16 oder 19 oder dem Ausgangsglas gemäß einem der Ansprüche 17 bis 19 durch Verpressen oder maschinelle Bearbeitung die Form der gewünschten dentalen Restauration gegeben wird.
